# EUROPEAN PATENT SPECIFICATION

(11) **EP 3 308 852 B1**
(45) Date of publication and mention of the grant of the patent: **02.12.2020**
(21) Application number: 16193281.9
(22) Date of filing: 11.10.2016
(51) Int. Cl.: B01J 19/00

(54) **APPARATUS FOR THE SYNTHESIS OF RADIOPHARMACEUTICAL PRODUCTS**
VORRICHTUNG ZUR SYNTHESE VON RADIOPHARMAZEUTISCHEN PRODUKTEN
APPAREIL DE SYNTHÈSE DE PRODUITS RADIOPHARMACEUTIQUES

(43) Date of publication of application: 18.04.2018
(73) Proprietor: Ion Beam Applications, 1348 Louvain-la-Neuve (BE)
(72) Inventor: GUYETTE, Michel, 1348 Louvain-la-Neuve (BE); COMOR, Jozef, 11070 Belgrade (RS)
(74) Representative: ABYOO

(56) References cited:
- EP-A1- 2 022 736
- WO-A1-2014/160799
- WO-A1-2016/146686
- WO-A1-2016/146693
- US-A1- 2006 245 980
- US-A1- 2013 170 931

## Description

### Field of the invention

The invention relates to a device for synthesis of radiopharmaceutical products from radioactive compounds and/or chemical reagents located on cassettes or disposable cassettes.

The invention relates more specifically to a device for synthesis of radiopharmaceutical products, said device being able to perform multiple runs to produce radiopharmaceutical products, without human intervention.

### Description of prior art

In diagnostic modalities, such as Positron Emission Tomography (PET), Single Photon Emission Tomography (SPECT), and therapeutic applications radiopharmaceutical or radiochemical compounds are used, which are labelled by means of radioactive elements, such as ¹¹C, ¹³N, ¹⁵O, ¹⁸F, ⁶⁸Ga, ¹⁷⁷Lu, ⁹⁰Y, ⁸⁹Zr, ⁶⁴Cu, ¹²⁴I, ¹²³I and many others for imaging and therapeutic purposes. The synthesis of these radiopharmaceutical compounds is performed in apparatuses that enable the different chemical compounds located on a cassette to be brought into contact and heated, when applicable, during synthesis reactions.

During the synthesis process, four major issues need to be addressed. The first issue concerns the handling of the compounds. The compounds comprise of radioactive substances, which may be harmful for the humans that handle the synthesis apparatus. To avoid any risk associated with the handling of radioactive products, the apparatuses are generally placed in a confined and shielded environment. These apparatuses are driven by an automaton means, which commands the various operations enabling the synthesis, including the reaction, mixing, transfer, heating, and purification stages. The automation of these apparatuses reduces human error and human exposure to radioactivity.

The second major issue concerns the quality and purity of the synthetized compounds. These products are injected into human beings for diagnostic or therapeutic applications. Therefore, to avoid any contamination issues or incorrect dosage of the reaction compounds, cassettes comprising all the reaction compounds in pre-metered bottles have been developed. Such cassettes allow making a single run to produce a radiopharmaceutical compound or substance.

The third major issue in this technical field concerns the half-life of the synthetized products (for example, products containing ¹⁸F, ⁶⁸Ga, etc.). Generally, radiopharmaceutical products have a short half-life and cannot be stored for later use. It is therefore necessary to produce radiopharmaceuticals at regular intervals and to make a more effective use of the apparatus during the day. It would be advantageous that syntheses could be performed successively ("back to back" runs). The compact automated synthesizers have a single interface between the cassette and the synthesizer. Moreover, cassettes are often disposable allowing a limited number of syntheses runs. Hence, after a limited number of syntheses runs occur, the human operator must open the confined and shielded environment, remove the cassettes from the apparatus, and add new cassettes in order to start production again. This can be dangerous, due to the radioactivity surrounding the synthesis apparatus. Moreover, this can lead to human errors. The less the human operators intervene during the synthesis process, the lower is the probability of making mistakes or introducing contaminations. This issue is currently tackled by existing devices in an unsatisfactory manner. One current solution requires more than one synthesis machine within the limited shielded environment, while the space available is constraint. One other solution is the re-use of disposable cassettes designated for a single-run, requiring a washing procedure, and jeopardizing the overall quality of the final product. Finally, due to the particularly short half-life of some of the isotopes incorporated into the radiopharmaceutical compounds, it may be necessary to avoid any loss of time for producing a needed product.

The fourth issue concerns the flexibility of use of the synthesis apparatus. Currently, with a single synthesis apparatus, it is not possible to program and synthetize a range of different radiopharmaceutical compounds without human intervention. When different radiopharmaceutical compounds need to be synthetized, and after a fist run, an operator must manually add a new and different cassette to the apparatus for the synthesis of a new product.

Various apparatuses have been developed during the last years, but not a single one addresses the four major issues mentioned above in a satisfactory way. As example, the European patent n° 1343533 and the American patent n° 7235216 disclose an apparatus and a cassette creating a reduced risk of contamination or incorrect dosages. The apparatus may comprise a lead chamber, to reduce safety risks for the human handling of the synthesis processes. Unfortunately, when multiple runs must be performed in a short interval, the above mentioned problems are not resolved. The loading of the cassette onto the synthesis apparatus has to be performed manually by an operator, exposing the operator to the residual radioactivity when he opens the shielded environment.

The document US2013/170931 describes an apparatus for manufacturing radiopharmaceutical products from reagents, this apparatus comprising a synthesis device and a loading device on which chemical systems in the form of a plurality of cassettes comprising the reagents may be mounted. D1 also discloses that the synthesis device comprises an interface and mechanical means able to act on the transfer means of the cassettes when the cassettes are connected to the interface but also that the apparatus comprises a shifting means able to successively shift the plurality of cassettes from a storage position to a connected position onto the interface. Finally, D1 also discloses that the synthesis device comprises ejecting means able to eject a cassette connected to the interface.

### Summary of the invention

To, at least partially, solve the problems of the state of the art, it is an object of the invention to provide a synthesis apparatus able to perform a plurality of synthesis runs in a safe and secured manner, without any human intervention during the plurality of synthesis. It is an object of the invention to provide an apparatus for the synthesis of radioactive products, especially radiopharmaceutical products, from radioactive elements, which can be used in a safe manner. Once a human has prepared the apparatus for multiple synthesis runs, the apparatus, according to the invention, is able to perform multiple runs successively without the need of human intervention. It is likewise an object of the present invention to provide an apparatus which minimizes the risk of contamination of the synthetized products, while the safety of humans handling the synthesis product is assured. While providing multiple consecutive synthesis runs, the current invention provides the feature to synthesize different products consecutively with the same apparatus, without human intervention between the consecutive runs. Consecutive runs can result in different products depending on which cassettes have been loaded on the loader.

The invention is defined by the independent claim. The dependent claims define advantageous embodiments.

According to the invention, an apparatus is provided for manufacturing of radiopharmaceutical products from reagents, the apparatus comprising a synthesis device and a loading device on which chemical systems in the form of a plurality of cassettes may be mounted, with the cassettes comprising a means for transferring the chemical reagents. The synthesis device contains an interface and mechanical means able to act on the transfer of the cassettes when the cassettes are connected to the interface.

The apparatus comprises a shifting means able to successively shift the plurality of cassettes from a storage position to a connected position onto the interface, and the synthesis device furthermore comprises an ejecting means able to eject a disposable cassette connected to the interface. The shifting means comprises:
- a transfer means able to successively transfer, in a first movement, the cassettes from the storage position to a loading position located on or near the interface, and
- a connecting means able to securely connect, in a second movement, the cassettes to the interface,
   both the transfer means and the connecting means being a piston or a hydraulic cylinder actuated by an automaton co-operating with a computer.

The apparatus is characterized in that the shifting means is able to place a positioning wedge on the connecting means when the shifting means is shifting a cassette from the storage position to the loading position.

Indeed, with an apparatus according to the invention, the four major issues mentioned above are at least partially tackled. The whole apparatus may be placed in a safe and shielded environment, or the whole apparatus itself may be shielded to avoid any emission of radioactivity outside of the synthesis apparatus. The plurality of runs is performed within cassettes that comprise for each run the necessary reagents for the synthesis of the desired radiopharmaceutical compounds. No human intervention is necessary in between runs, leading to a safe production of the radioactive compounds and no harmful radiation exposure to the operator(s). Another major advantage of the present invention is that multiple synthesis of the same radiopharmaceutical compound may be performed, when all of the cassettes are of the same kind. In addition, it is also possible to perform syntheses of a range of different radiopharmaceutical compounds when the cassettes are of a different kind. Hence, according to the needs of the patients for the radiopharmaceutical compounds, the apparatus allows the production of various compounds in correlation with these needs. If a number of patients need to receive the same radiopharmaceutical compound, a sufficient quantity may be produced without human intervention once the apparatus is loaded with the cassettes. On the other hand, when a number of patients need to receive different radiopharmaceutical compounds, a variety of these compounds may be produced without human intervention. It is therefore possible to produce the same or different radioactive compounds without human intervention. With the apparatus, according to the invention, the loading of each new cassette onto the synthesis apparatus between synthesis runs is automatic.

The apparatus according to the invention has shifting means comprising a transfer means able to successively transfer the cassettes from the storage position to a loading position located near the interface, and the apparatus comprises means able to securely connect the cassettes to the interface.

According to the invention, the connection of each cassette to the interface located on the synthesis device is secured, leading to a more reliable apparatus, avoiding any misconnection between the cassette and the interface.

The apparatus according to the invention comprises a shifting means able to dispose a positioning wedge on the securely connecting means when the shifting means shifts a cassette from the storage position to the loading position.

According to the invention, the interaction between the shifting means and the securely connecting means ensure an even more reliable positioning and connection of the cassette.

### Short description of the drawings

These and further aspects of the invention will be explained in greater detail by way of examples and with reference to the accompanying drawings in which:
- Fig.1: schematically shows an apparatus according to one embodiment of the invention wherein the apparatus is loaded with several cassettes and a cassette is connected to the interface.
- Fig.2: schematically shows the apparatus of fig.1 wherein the cassettes are all disposed on the loading device.
- Fig.3: schematically shows the apparatus of fig. 1 wherein a placing means is illustrated.
- Fig.4: schematically shows the apparatus of fig.1 wherein the shifting means is illustrated.
- Fig.5: schematically shows the apparatus of fig.2 wherein the shifting means is illustrated.

The drawings of the figures are neither drawn to scale nor proportioned. Generally, similar or identical components are denoted by the same reference numbers in the figures.

### Detailed description of embodiments of the invention

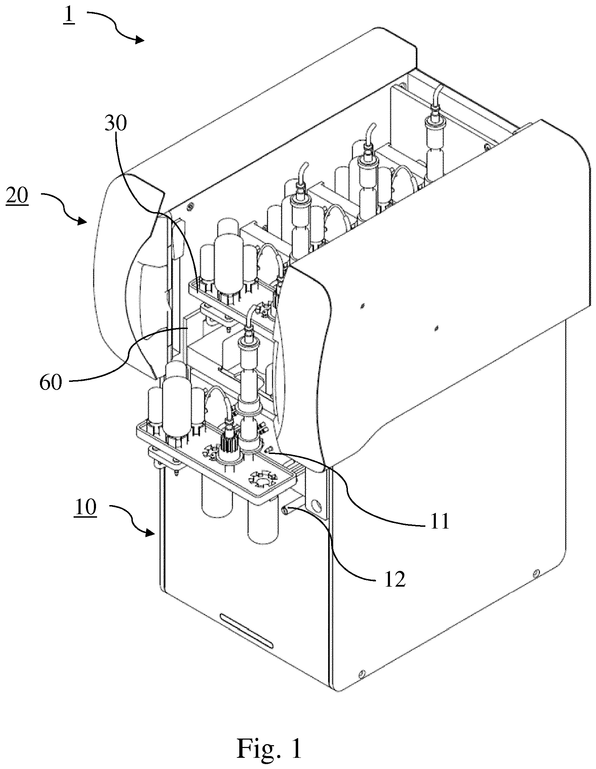

As illustrated in **Fig.1****,** the apparatus (1) according to the present invention comprises a synthesis device (10) and a loading device (20). The apparatus is able to produce radiopharmaceutical products from reagents, preferably chemical reagents, when a cassette (30) is placed or fixed on an interface (11) and the apparatus (1) is in operation. The cassette may be a reusable cassette or a disposable cassette.

A cassette (30), also known as module, is a removable cassette well known by a man of the art , which may be reusable, washable, or preferably disposable after a single or several syntheses runs. In a more preferred embodiment, the cassette is a disposable cassette, and more preferably a single use disposable cassette. Such cassette is generally in the shape of a support means and may contain chemical reagents, one or several reaction compartments, transfer means between the chemical reagents and the reaction compartments. Such cassette is described for example in document EP1343533.

The cassette (30), on which transfer, reaction and purification functions are performed and the chemical reagents and reaction compartments are arranged, is intended for association with the synthesis device (10) on the interface (11).

The interface (11) may be a fixed module located on the synthesis device (10), and wherein a plurality of mechanical means are arranged, these mechanical means being able to cooperate with the transfer means present on the disposable cassette (30) to allow the transfer of the reagents from the various reagents to the various reaction compartments. The various operations for synthesis of pharmaceutical products, including the actuation of these mechanical means, are commanded via an automaton. In other words, the apparatus (1), and more specifically the synthesis device (10), may be linked to an automaton which commands the various operations enabling the performance of the synthesis, comprising reaction and heating stages; which are associated with transfers of the reagents. This is also well known by a man of the art, and is described in document EP 1343533.

As illustrated on the Fig.1, the loading device (20) may be mounted over the synthesis device (10). It should nevertheless be noted that the loading device (20) may be mounted on other parts of, next to the synthesis device (10), for example on one side of the synthesis device. In another embodiment of the invention, the loading device (20) can even be placed outside of the shielded environment. In a preferable embodiment, the loading device (20) is mounted on the top of the synthesis device (10). The synthesis device (20) could be self-shielded or placed in a shielded environment (also known as a "hot cell"). In an alternative embodiment, the apparatus (1) comprising the synthesis device (10) and the loading device (20) is self-shielded (i.e. surrounded by lead plates or enclosed within a lead shield). In other words, the synthesis device or the entire apparatus are enclosed within a shield reducing radiation emission outside of the shielded synthesis device or shielded apparatus.

A plurality of cassettes (30) may be mounted on the loading device (20). As illustrated on Fig.1, the plurality of cassettes may be mounted on a rack (60) in a linear disposition, the cassettes being parallel to each other. The rack may have the form of two horizontal strips or bars on which the cassettes are placed. It should be noted that other disposition may be contemplated. For example, the plurality of cassettes could be disposed on a carousel. By plurality of cassettes, it should be understand that at least two cassettes may be placed on the loading device, in a more preferred embodiment, at least three cassettes may be disposed on the loading device and, in a more preferred embodiment, at least four cassettes may be disposed on the loading device (20). Of course, the loading device (20) may have a different shape. For example, the loading device may be a circular platform wherein the cassettes are not parallel to each other, but mounted on the circular platform like spokes.

It should also be noted that the plurality of cassette does not have to be identical regarding their chemical reagents content and/or their reaction compartments. The external edge of the plurality of cassettes (30) should of course have the same shape to cooperate with the loading device (20), but the content of the reagents may be different, for example when a diversity of radiopharmaceutical compounds have to be produced. Of course, the plurality of cassettes (30) could be identical regarding their content when a single radiopharmaceutical compound has to be produced by the synthesis device (10).

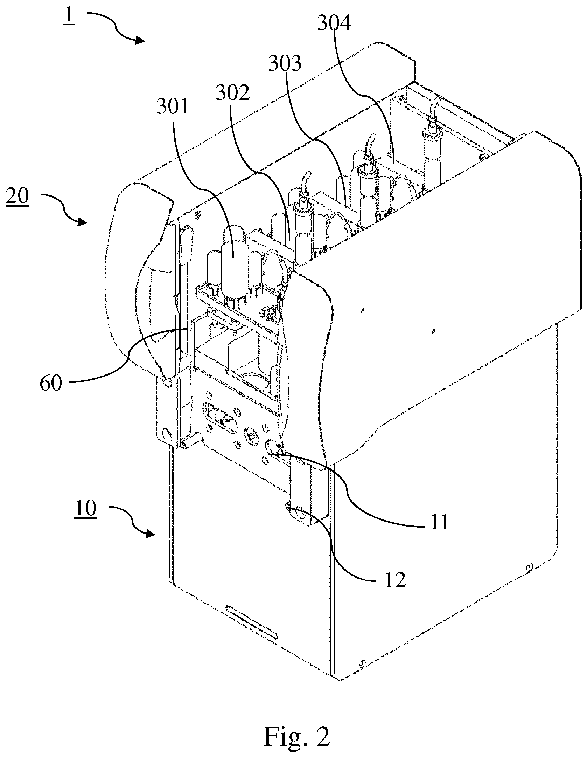

When a person is preparing the apparatus according to the invention for multiple runs to synthesize different products, he just needs to place/put each cassette on the loading device, in the order intended for synthesis of the various radiopharmaceutical products. Once the cassettes are placed on the loading device (20), no direct human intervention is needed for the loading, shifting, connection or ejection of each cassette. When a cassette is placed on the loading device (20), the cassette is defined as being on its stacking or storage position. This is illustrated on Fig.2.

The apparatus (1) comprises means able to successively shift the plurality of cassettes (30) from a storage position to a connected position onto the interface (11). By shifting, it should be understood the movement of each cassette from a position where the cassette is placed on the loading device (20) in its storage position, to the position where the cassette is functionally connected to the synthesis device (10) by way of the interface (11) (the connected position). The functional connection between the synthesis device (10) and a cassette is illustrated on Fig.1. The shift from a storage position to a connected position onto the interface (11) is performed by at least one shifting means (32). This shifting means may interact with an edge of the cassette, and shift it from its storage position to the connected position. The function of the shifting means (32) is to successively move each cassette from its storage position (illustrated on Fig.2) to its connected position with the interface (11), illustrated on Fig.1. The shifting means (32) may be present on the loading device (20) or on the synthesis device (10). In a preferred embodiment, the shifting means are on the loading device (20).

A variety of shifting means (32) may be contemplated. For example, the shifting means may be a piston or a hydraulic cylinder controlled by an automaton. When the piston or a hydraulic cylinder is activated, it shifts the cassette from its storage position to the connected position. It should be noted that the shifting movement may be any kind of movement, like a rotation of the cassette, a translation of the cassettes, a plurality of translations of the cassettes, or a combination between rotation and translation.

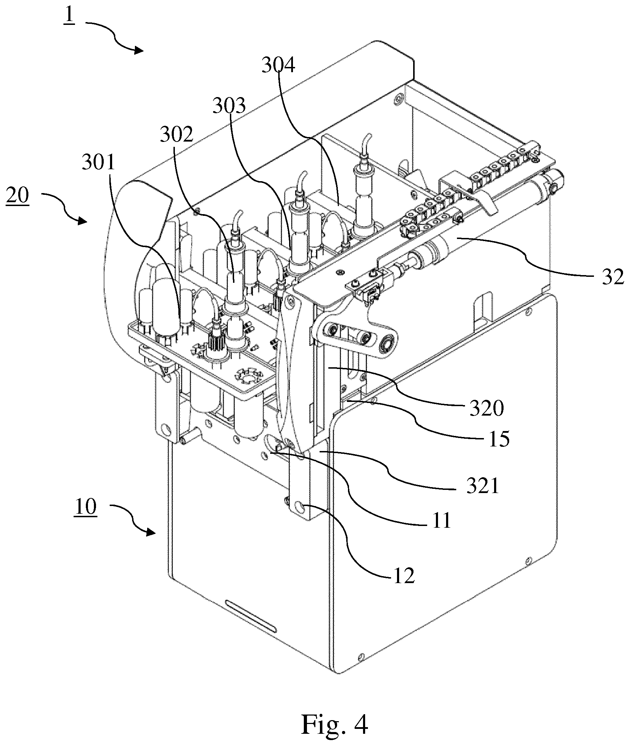

The shifting means may be present on the synthesis device (10) or on the loading device (20). In a preferred embodiment, the shifting means are located on the loading device (20) as illustrated for example on Fig.4.

When the cassette is on its connected position, as illustrated on Fig.1, the synthesis of the radiopharmaceutical products may proceed. The synthesis device (10) interacts with the cassette connected to the interface (11) by way of mechanical means as explained in [0022]. The whole interaction between the synthesis device (20) and the transfer means on the cassette may be commanded by an automaton.

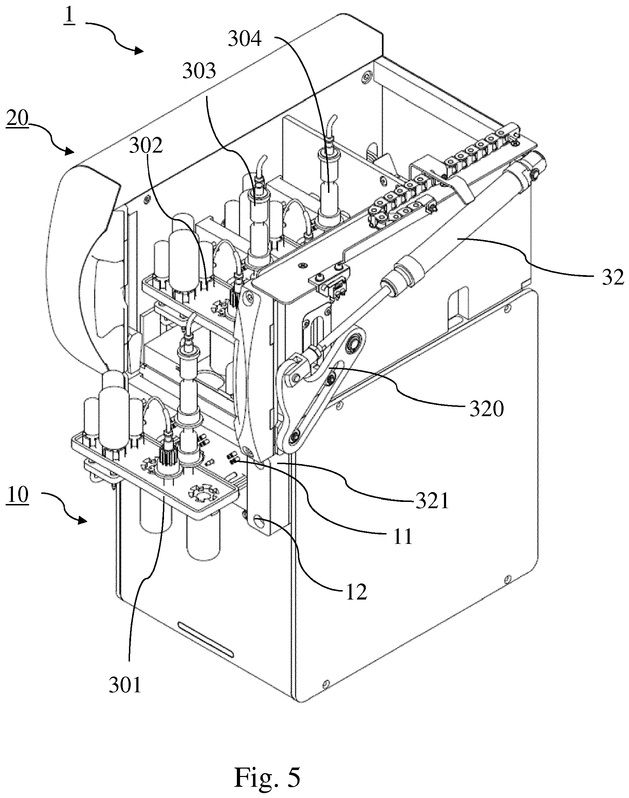

When the synthesis is over, the cassette connected to the interface (11) is ejected. To this end, the synthesis device (10) comprises of means able to eject the cassette connected to the interface (11). This is illustrated on Fig. 5. As illustrated, an ejecting means (12) is located near the interface (11). When the synthesis run is over, the ejecting means is actuated and the cassette is removed from the interface, and may be discarded into a shielded bin. The ejecting means may, for example, be a piston or a hydraulic cylinder that, when actuated, allows to take down or unhook the cassette from the interface (11). The cassette may then fall into shielded bin. Once the cassette is ejected, the ejecting means may return to its original position.

Once the cassette used for a synthesis run is ejected from the interface (11), another cassette located on the loading device (20) on its storage position may be shifted from this position to the connected position of the interface (11).

Accordingly, as long as a cassette is present on the loading device (20), a new synthesis run may be performed after the ejection of the previous cassette, without any human intervention.

The shifting means (32) comprises a transfer means (320) and a connecting means (321). In this embodiment, the shifting of the cassette from its storage position to the connected position comprises of two movements. The first movement is the movement of the cassette from its storage position, illustrated on Fig.2, to a loading position, illustrated on Fig.4. The loading position is a position located nearby the interface (11) but the cassette is not connected to the interface (11). This movement is achieved by the transfer means (320). The transfer means may, for example, be a piston or a hydraulic cylinder. When the piston or the hydraulic cylinder is activated, it moves the cassette from its loading position to the connected position. It should be noted that this movement may be any kind of movement, like a rotation of the cassette, a translation of the cassettes, a plurality of translations of the cassettes, or a combination between rotation and translation. According to this preferred embodiment, the second movement is the movement of the cassette from the loading position to the connected position, as illustrated respectively on Fig.2 and Fig.1. This movement is achieved by the connecting means. It allows to carefully secure the connection between the cassette and the interface (11), reducing risks of incorrect connection between the cassette and the interface, to avoid damages to the cassette and/or to the interface (i.e. the mechanical means located on the interface) to ensure the synthesis process will take place effectively. As an example, this tight and accurate movement of the connecting means that ensure correct interaction between the cassette and the interface (11) avoids any leaks of the reagents after connection.

The connecting means may, for example, be a piston or a hydraulic cylinder that translates the cassette from the loading position to the connected position, as illustrated on Fig.4. In a preferred embodiment, the movement from the loading position to the connected position is a single translation movement. This simple movement ensures an easy and accurate connection between the interface (11) and the cassettes.

In a preferred embodiment of the invention, the connecting means are located on the synthesis device (20), and still in a preferred embodiment, near the interface (11). In a more preferred embodiment, the ejecting means and the connecting means are able to eject a cassette when it is connected to the interface (11) and able to securely connect the cassette when it is in the loading position. This means may be a piston or a hydraulic cylinder. In a more preferred embodiment, this same means is a double acting hydraulic cylinder.

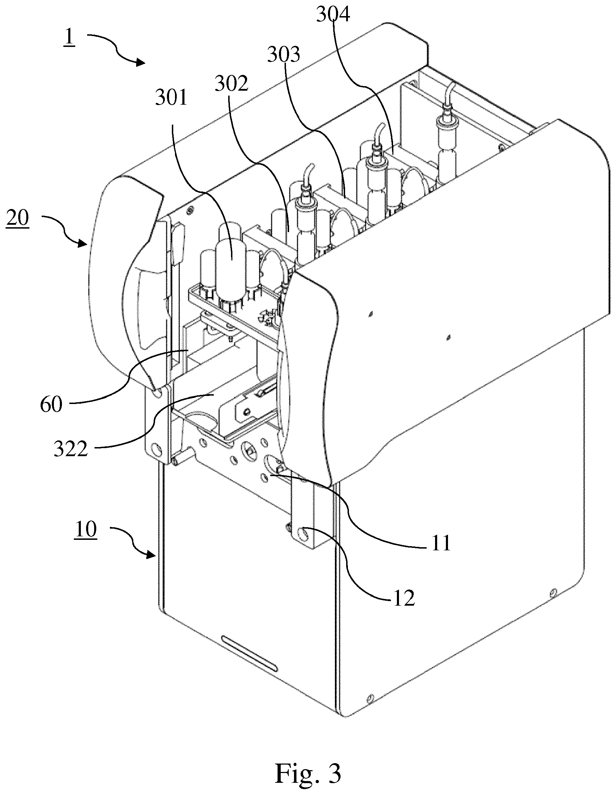

In a preferred embodiment of the invention, the shifting means (32) also comprises placing means (322). This is for example illustrated on Fig.3. The placing means (322) is able to successively place the cassettes from a stacking position to its storage position, both potentially located on the loading device (20). In the more general embodiment of the invention, these two positions may be the same. In this particular embodiment, each cassette is successively moved from its stacking position, i.e. the position where it has been placed on the loading device (20) before the apparatus begins to run a plurality of syntheses, to the storage position, already defined earlier, i.e. the position where the cassette are shifted through the connected position. As an example, illustrated on Fig.2, the cassettes are placed parallel to each other on a rack. The first cassette (301) is on its storage position, while the three other cassettes (302, 303, 304) are on their stacking position. Once the first cassette (301) shifts from the storage position to the connected position, the second cassette (302) moves from its stacking position to the storage position. This movement may occur during the shifting movement of the first cassette, but it may also occur after the first cassette (301) has been ejected by the ejecting means (12), as illustrated on Fig.4. As illustrated on Fig.1 and 4, the various cassettes may have a plurality of stacking positions as they are gradually moved on the rack through the storage position.

The placing means (322) that allows the movement of the cassette from a stacking position to a storage position may for example be a piston or hydraulic cylinder linked to the rack or to each cassette, for example with a pushing means like a mechanical finger or a grasping means like a jaw. In a preferred embodiment of the invention, the placing means (322) are able to linearly move the cassettes from their stacking position to the storage position. As an example, the loading device (20) may comprise of a rail on which the cassettes are placed on their storage position. The rail is gradually moved through the stacking position by the placing means, pushing the disposable cassettes from their stacking position to the storage position.

In a preferred embodiment of the invention, the shifting means is able to linearly shift the cassette from its storage position to its connected position. Alternatively, when the shifting means comprise a connecting means, the shifting means is able to linearly shift the cassette form its storage position to the loading position, and the connecting means may be able to linearly move the cassette from the loading position to the connected position. These linear movements are simple movement, which do not require complex arrangement between the various means that are able to move the cassette from one place to another. It ensures a more robust device, and allows great movement accuracy. As an example, the shifting means, and the connecting means when present, may both be piston or hydraulic cylinder, preferably actuated by an automaton co-operating with a computer.

According to the invention, the shifting means (32) is furthermore able to place a positioning wedge (15) on the connecting means when the shifting means (32) is shifting a cassette from its storage position to the loading position. This embodiment requires that the connecting means (321) is present. The wedge allows the finely positioning of a part of the connecting means (321) that will be in interaction with the cassette. The interaction between the cassette and the connecting means (321) is therefore more secured and easier. It ensures that the connection movement operated by the connecting means is a precise and accurate movement, allowing a good interaction between the cassette and the interface (11) after the connection movement. This embodiment is for example illustrated on Fig.5. As one can see, both shifting means (32) and connecting means (321) are hydraulic cylinders. The connecting means (321) comprise a first movable part that is able to interact with the cassette, and a second fixed part. The first part and the second part are linked by the hydraulic cylinder that allows the movement of the first part. When the shifting means is shifting the cassette to the loading position, it also places the positioning wedge (15) between the first part and the second part. The connecting means may then be retracted, i.e. the hydraulic cylinder is actuated to move the first part through the second part. The wedge is dimensioned to allow the first part to be placed exactly where it is able to interact correctly with the cassette once it reached the loading position. When the cassette is located on the loading position, the connecting means interacts with the cassette in a secure manner, avoiding any damages to the cassette. The shifting means may then be actuated backwards removing the wedge from the connecting means. Finally, the connecting means may be actuated to connect the cassette to the interface (11).

In a more preferred embodiment, the connecting means may furthermore comprise interacting means able to interact with the positioning means located on the cassette. These interacting means allows an improved interaction between the cassettes and the connecting means (321), improving even more the accuracy of the movement of the cassette from the loading position to the connected position. A positioning means may be for example a groove able to interact with a rod located on the disposable cassette. Of course, a groove may be located on the cassette while the positioning means is a rod. Similar means are also contemplated by the present invention.

In a preferred embodiment of the invention, the various means for moving the cassette from any position to another position are actuated by an automaton co-operating with a computer. In other words, the various moving means are linked to an automaton co-operating with a computer, said automaton being able to command the operations enabling the movements of the plurality of cassettes. This allows an automatic actuation of the different means to ensure a correct sequence of actuation of each means to move successively each cassette located on the loading device to the interface, and eject it after each synthesis run. The computer may of course comprise dedicated software to program these actuation sequences.

In another aspect of the invention, the apparatus furthermore comprises a plurality of cassette. Each cassette comprises at least one shoulder able to fit into grooves located on the loading device (20), the placing means (322) being able to translate at least one cassette through the grooves. This ensures a correct movement of the cassette from a stacking position to the storage position or to another stacking position.

The cassette may furthermore comprise dedicated means able to interact selectively or universally with the various moving means (the shifting means, the connecting means, the placing means, the ejecting means). By selectively interacting, it should be understood that a plurality of different means each able to cooperate with only a single moving means are located on the cassette; while by universally interacting, it should be understood that a single means located on the cassette is able to cooperate with all the moving means. As an example, the moving means may comprise grooves and the cassette may comprise a rod. The various moving means are all able to translate the cassette through the groove for moving it from one location to another location *via* its interaction with the rod located on the cassette. Other means may also be considered by the present invention, like a plurality of grasping means.

In a preferred embodiment of the invention, the shifting means (32) furthermore comprises a pushing means like a mechanical finger or a grasping means like a jaw, a hooking means or a seizing means able to grasp, hook or seize a disposable cassette. These means are able to drag or -push the cassette through the connected positon or the loading position when the shifting means is actuated. This allows an easier movement of the cassette. For example, when the loading device (20) is located on the top of the synthesis device, the grasping means may help to properly move the disposable cassette by transmitting a more important moving force to the disposable cassette. As a preferred embodiment, these means are a pushing means, like a finger, that pushes downward the disposable cassette through its connection position.

## Claims

1. An apparatus (1) for manufacturing radiopharmaceutical products from reagents, the apparatus (1) comprising a synthesis device (10) and a loading device (20) on which chemical systems in the form of a plurality of cassettes (30) comprising the reagents may be mounted, said cassettes (30) comprising means for transferring the chemical reagents,
the synthesis device (10) comprising an interface (11) and mechanical means able to act on the transfer means of the cassettes when the cassettes (30) are connected to the interface (11),
the apparatus (1) comprising a shifting means (32) able to successively shift the plurality of cassettes (30) from a storage position to a connected position onto the interface (11),
and the synthesis device (10) furthermore comprising ejecting means (12) able to eject a cassette connected to the interface (11),
said shifting means (32) comprising:
- a transfer means (320) able to successively transfer, in a first movement, the cassettes (30) from the storage position to a loading position located on or near the interface (11), and
- a connecting means (321) able to securely connect, in a second movement, the cassettes (30) to the interface (11),
both the transfer means (320) and the connecting means (321) being a piston or a hydraulic cylinder actuated by an automaton co-operating with a computer,
the apparatus (1) being **characterized in that** the shifting means (32) is able to place a positioning wedge (15) on the connecting means (321) when the shifting means (32) is shifting a cassette from the storage position to the loading position.

2. An apparatus (1) according to claim 1, **characterized in that** the shifting means (32) are located at least partially on the loading device (20).

3. An apparatus (1) according to claim 1, **characterized in that** the connecting means (321) are located on the synthesis device (10).

4. An apparatus (1) according to any one of the previous claims, **characterized in that** the loading device (20) comprises placing means (322) able to successively place the cassettes (30) from a stacking position to the storage position.

5. An apparatus (1) according to claim 4, **characterized in that** the placing means (322) are able to linearly shift the cassettes (30) from their stacking position to the storage position.

6. An apparatus (1) according to any one of the previous claims, **characterized in that** the shifting means (32) are able to linearly shift the cassettes (30) from the storage position to the connected position, and/or from the storage position to the loading position.

7. An apparatus (1) according to any one of the previous claims, **characterized in that** the ejecting means (11) and the connecting means (321) are a same means.

8. An apparatus (1) according to claim 7, **characterized in that** the connecting means (321) comprises interacting means able to interact with positioning means located onto the cassettes (30) for positioning the cassettes (30).

9. An apparatus (1) according to any one of the previous claims, **characterized in that** the synthesis device (10) is self-shielded or located in a shielded environment, and more preferably **in that** the synthesis device (10) and the loading device (20) are self-shielded or located in a shielded environment.

10. An apparatus (1) according to any one of the previous claims, **characterized in that** the shifting means (32), the ejecting means (12), and when present the transfer means (320), the connecting means (321), the placing means (322), are linked to an automaton co-operating with a computer, said automaton being able to command the operations enabling the shifting, the placing, the transfer, the connection and/or the ejection of the plurality of cassettes (30).

11. An apparatus (1) according to claim 4, the apparatus furthermore comprising the cassettes (30), and **characterized in that** each cassette comprises at least one shoulder able to fit into grooves located on the loading device (20) and/or the synthesis device (10), the placing means (322) being able to translate the cassettes by moving them through the grooves.

12. An apparatus according to any one of the previous claims, **characterized in that** the shifting means (32) furthermore comprises a pushing means or grasping means able to guide or grasp a cassette, with the pushing or grasping means being able to move a cassette through the connected position or the loading position when shifting means is actuated.

## Patentansprüche

1. Vorrichtung (1) zum Herstellen von radiopharmazeutischen Produkten von Reagenzien, wobei die Vorrichtung (1) ein Synthesegerät (10) und ein Ladegerät (20) umfasst, auf dem chemische Systeme in der Form einer Vielzahl von die Reagenzien umfassenden Kassetten (30) montiert sein können, wobei die genannten Kassetten (30) Mittel zum Übertragen der chemischen Reagenzien umfassen,
wobei das Synthesegerät (10) eine Schnittstelle (11) und mechanische Mittel umfasst, die geeignet sind, auf dem Übertragungsmittel der Kassetten zu wirken, wenn die Kassetten (30) an die Schnittstelle (11) angeschlossen sind,
wobei die Vorrichtung (1) ein Versetzungsmittel (32) umfasst, das geeignet ist, die Vielzahl von Kassetten (30) sukzessive von einer Speicherposition in eine angeschlossene Position auf der Schnittstelle (11) zu versetzen,
und wobei das Synthesegerät (10) weiterhin Auswurfmittel (12) umfasst, die geeignet sind, eine an die Schnittstelle (11) angeschlossene Kassette auszuwerfen,
wobei das genannte Versetzungsmittel (32) umfasst:
- ein Übertragungsmittel, das geeignet ist, um in einer ersten Bewegung die Kassette (30) von der Speicherposition in eine auf oder in der Nähe der Schnittstelle (11) angeordnete Ladeposition sukzessive zu übertragen, und
- ein Anschlussmittel (321), das geeignet ist, die Kassetten (30) in einer zweiten Bewegung sicher an die Schnittstelle (11) anzuschließen,
wobei das Übertragungsmittel (320) und das Anschlussmittel (321) ein Kolben oder ein hydraulischer Zylinder sind, die durch einen mit einem Computer zusammenwirkenden Automaten betätigt sind, wobei die Vorrichtung (1) **dadurch gekennzeichnet ist, dass** das Versetzungsmittel (32) geeignet ist, einen Positionierungskeil (15) auf dem Anschlussmittel (321) zu positionieren, wenn das Versetzungsmittel (32) eine Kassette von der Speicherposition zur Ladeposition versetzt.

2. Vorrichtung (1) gemäß Anspruch 1, **dadurch gekennzeichnet, dass** das Versetzungsmittel (32) wenigstens teilweise auf dem Ladegerät (20) angeordnet ist.

3. Vorrichtung (1) gemäß Anspruch 1, **dadurch gekennzeichnet, dass** das Anschlussmittel (321) auf dem Synthesegerät (10) angeordnet ist.

4. Vorrichtung (1) gemäß irgendeinem der voranstehenden Ansprüche, **dadurch gekennzeichnet, dass** das Ladegerät (20) Anordnungsmittel (322) umfasst, die zum sukzessiven Platzieren der Kassetten (30) von einer Stapelposition in die Lagerposition geeignet ist.

5. Vorrichtung (1) gemäß Anspruch 4, **dadurch gekennzeichnet, dass** die Anordnungsmittel (322) geeignet sind, um die Kassetten (30) von ihrer Stapelposition in die Speicherposition linear zu versetzen.

6. Vorrichtung (1) gemäß irgendeinem der voranstehenden Ansprüche, **dadurch gekennzeichnet, dass** das Versetzungsmittel (32) zum linearen Versetzen der Kassetten (30) von der Speicherposition zur angeschlossenen Position und / oder von der Speicherposition zur Ladeposition geeignet ist.

7. Vorrichtung (1) gemäß irgendeinem der voranstehenden Ansprüche, **dadurch gekennzeichnet, dass** das Auswurfmittel (11) und das Anschlussmittel (321) ein und dieselben Mittel sind.

8. Vorrichtung (1) gemäß Anspruch 7, **dadurch gekennzeichnet, dass** das Anschlussmittel (321) zusammenwirkende Mittel umfasst, die geeignet sind, mit Positionierungsmitteln zusammenzuwirken, die auf den Kassetten (30) zum Positionieren der Kassetten (30) angeordnet sind.

9. Vorrichtung (1) gemäß irgendeinem der voranstehenden Ansprüche, **dadurch gekennzeichnet, dass** das Synthesegerät (10) selbst-abgeschirmt oder in einer abgeschirmten Umgebung angeordnet ist und weiter bevorzugt darin, dass das Synthesegerät (10) und das Ladegerät (20) selbst-abgeschirmt oder in einer abschirmten Umgebung sind.

10. Vorrichtung (1) gemäß irgendeinem der voranstehenden Ansprüche, **dadurch gekennzeichnet, dass** das Versetzungsmittel (32), das Auswurfmittel (12) und, sofern vorhanden, das Übertragungsmittel (320), das Anschlussmittel (321), das Anordnungsmittel (322) mit einem mit einem Computer zusammenwirkenden Automaten verbunden sind, wobei der genannte Automat geeignet ist, die Arbeitsabläufe zu steuern, die das Versetzen, das Anordnen, das Übertragen, das Anschließen und / oder das Auswerfen der Vielzahl von Kassetten (30) ermöglichen.

11. Vorrichtung (1) gemäß Anspruch 4, wobei die Vorrichtung weiterhin die Kassetten (30) umfasst und **dadurch gekennzeichnet ist, dass** jede Kassette wenigstens eine Schulter umfasst, die geeignet ist, sich in Rillen einzupassen, die auf dem Ladegerät (20) und / oder dem Synthesegerät angeordnet sind, wobei die Anordnungsmittel (322) geeignet sind, die Kassetten durch deren Bewegen durch die Rillen umzusetzen.

12. Vorrichtung gemäß irgendeinem der voranstehenden Ansprüche, **dadurch gekennzeichnet, dass** das Versetzungsmittel (32) weiterhin ein Schubmittel oder Ergreifungsmittel umfasst, das geeignet ist, eine Kassette zu führen oder zu ergreifen, wobei das Schub- oder Ergreifungsmittel geeignet ist, eine Kassette durch die angeschlossene Position oder die Ladeposition zu bewegen, wenn das Versetzungsmittel betätigt ist.

## Revendications

1. Appareil (1) pour fabriquer des produits radio-pharmaceutiques à partir d'agents réactifs, l'appareil (1) comprenant un dispositif de synthèse (10) et un dispositif de chargement (20) sur lequel des systèmes chimiques se présentant sous la forme d'une pluralité de cassettes (30) contenant les agents réactifs peuvent être montés, lesdites cassettes (30) comprenant des moyens pour transférer les agents réactifs chimiques,
le dispositif de synthèse (1) présentant une interface (11) et des moyens mécaniques capables d'agir sur les moyens de transfert des cassettes lorsque les cassettes (30) sont connectées à l'interface (11),
l'appareil (1) comprenant des moyens de déplacement (32) capables de déplacer de façon successive la pluralité de cassettes (30) à partir d'une position de stockage dans une position connectée sur l'interface (11), et
le dispositif de synthèse (10) comprend en outre des moyens d'éjection (12) capables d'éjecter une cassette qui est connectée à l'interface (11),
lesdits moyens de déplacement (32) comprenant:
des moyens de transfert (320) capables de transférer de façon successive les cassettes (30) à partir de la position de stockage dans une position de chargement qui est située sur ou à proximité de l'interface (11), et
des moyens de connexion (321) capables de connecter de façon sûre, par l'intermédiaire d'un second déplacement, les cassettes (30) à l'interface (11),
les moyens de transfert (320) et les moyens de connexion (321) sont tous les deux un piston ou un cylindre hydraulique qui est actionné par un automate qui coopère avec un ordinateur,
l'appareil (1) étant **caractérisé en ce que** les moyens de déplacement (32) sont capables de placer une cale de positionnement (15) sur les moyens de connexion (321) lorsque les moyens de déplacement (32) déplacent une cassette à partir de la position de stockage dans la position de chargement.

2. Appareil (1) selon la revendication 1, **caractérisé en ce que** les moyens de déplacement (32) sont situés au moins partiellement sur le dispositif de chargement (20).

3. Appareil (1) selon la revendication 1, **caractérisé en ce que** les moyens de connexion (321) sont situés sur le dispositif de synthèse (10).

4. Appareil (1) selon l'une quelconque des revendications précédentes, **caractérisé en ce que** le dispositif de chargement (20) comprend des moyens de placement (322) capables de placer de façon successive les cassettes (30) à partir d'une position d'empilement dans la position de stockage.

5. Appareil (1) selon la revendication 4, **caractérisé en ce que** les moyens de placement (322) sont capables de déplacer les cassettes (30) de façon linéaire à partir de leur position d'empilement dans la position de stockage.

6. Appareil (1) selon l'une quelconque des revendications précédentes, **caractérisé en ce que** les moyens de déplacement (32) sont capables de déplacer les cassettes (30) de façon linéaire à partir de la position de stockage dans la position connectée, et/ou à partir de la position de stockage dans la position de chargement.

7. Appareil (1) selon l'une quelconque des revendications précédentes, **caractérisé en ce que** les moyens d'éjection (11) et les moyens de connexion (321) sont les mêmes moyens.

8. Appareil (1) selon la revendication 7, **caractérisé en ce que** les moyens de connexion (321) comprennent des moyens d'interaction capables d'interagir avec des moyens de positionnement qui sont situés sur les cassettes (30) pour positionner les cassettes (30).

9. Appareil (1) selon l'une quelconque des revendications précédentes, **caractérisé en ce que** le dispositif de synthèse (10) est auto-blindé ou est situé dans un environnement blindé, et plus préférablement **en ce que** le dispositif de synthèse (10) et le dispositif de chargement (20) sont auto-blindés ou sont situés dans un environnement blindé.

10. Appareil (1) selon l'une quelconque des revendications précédentes, **caractérisé en ce que** les moyens de déplacement (32), les moyens d'éjection (12), et lorsqu'ils sont présents les moyens de transfert (320), les moyens de connexion (321) et les moyens de placement (322), sont reliés à un automate qui coopère avec un ordinateur, ledit automate étant capable de commander les opérations qui permettent le déplacement, le placement, le transfert, la connexion et/ou l'éjection de la pluralité de cassettes (30).

11. Appareil (1) selon la revendication 4, l'appareil comprenant en outre les cassettes (30), et **caractérisé en ce que** chaque cassette comporte au moins un épaulement qui est capable de s'agencer dans des rainures qui sont situées sur le dispositif de chargement (20) et/ou sur le dispositif de synthèse (10), les moyens de placement (322) étant capables de déplacer les cassettes en les déplaçant à travers les rainures.

12. Appareil selon l'une quelconque des revendications précédentes, **caractérisé en ce que** les moyens de déplacement (32) comprennent en outre des moyens de poussée ou des moyens de saisie capables de guider ou de saisir une cassette, les moyens de poussée ou de saisie étant capables de déplacer une cassette à travers la position connectée ou la position de chargement lorsque les moyens de déplacement sont actionnés.
